# EUROPÄISCHE PATENTSCHRIFT

(11) **EP 3 285 824 B1**
(45) Veröffentlichungstag und Bekanntmachung des Hinweises auf die Patenterteilung: **25.12.2019**
(21) Anmeldenummer: 16717838.3
(22) Anmeldetag: 20.04.2016
(51) Int. Cl.: A61M 1/14

(54) **DIALYSEMASCHINE UND VERFAHREN ZUM BETRIEB EINES PNEUMATIKSYSTEMS EINER DIALYSEMASCHINE**
DIALYSIS MACHINE AND A METHOD FOR OPERATING A PNEUMATIC SYSTEM OF A DIALYSIS MACHINE
MACHINE À DIALYSE ET PROCÉDÉ POUR FAIRE FONCTIONNER UN SYSTÈME PNEUMATIQUE D'UNE MACHINE À DIALYSE

(30) Priorität: 22.04.2015 DE 102015005179
(43) Veröffentlichungstag der Anmeldung: 28.02.2018
(73) Patentinhaber: Fresenius Medical Care Deutschland GmbH, 61352 Bad Homburg (DE)
(72) Erfinder: MÜLLER, Ralf, 61350 Bad Homburg (DE); GRONAU, Sören, 65428 Rüsselsheim (DE)
(74) Vertreter: Herrmann, Uwe
(86) Internationale Anmeldenummer: PCT/EP2016/000642
(87) Internationale Veröffentlichungsnummer: WO 2016/169651

(56) Entgegenhaltungen:
- US-A1- 2011 120 302
- US-A1- 2013 025 692
- US-A1- 2013 028 788
- US-A1- 2014 174 542

## Beschreibung

Die vorliegende Erfindung betrifft eine Dialysemaschine mit einem Hydrauliksystem, das zur Bereitstellung der Dialyselösung oder eines Bestandteils der Dialyselösung bestimmt ist, wobei das Hydrauliksystem eine Entgasungspumpe zur Entgasung der Dialyselösung oder des Bestandteils der Dialyselösung aufweist, sowie mit einem Pneumatiksystem, in dem zum Betrieb einer Komponente des Dialysegerätes zumindest zeitweise Unterdruck herrscht.

Bei aus dem Stand der Technik bekannten Dialysemaschinen ist ein Hydrauliksystem vorgesehen, das beispielsweise Leitungen, Ventile, Pumpen etc. aufweist. Dieses Hydrauliksystem steht üblicherweise mit einem Wassereinlass in Verbindung und dient dazu, das Wasser, das zur Herstellung der Dialyselösung dient zu fördern sowie zu entgasen bzw. dazu dient, die fertige Dialyselösung herzustellen.

Zur Entgasung ist eine Entgasungspumpe vorgesehen, die üblicherweise in einem Entgasungsbehälter Unterdruck erzeugt, d.h. der Entgasungsbehälter befindet sich auf der Saugseite der Entgasungspumpe.

Durch die Unterdruckerzeugung im Entgasungsbehälter wird aus dem Wasser oder einer sonstigen Flüssigkeit Gas abgetrennt, das abgeleitet wird. Das auf diese Weise entgaste Wasser kann mittels der Entgasungspumpe zum Beispiel in ein Mischsystem zur Herstellung der Dialyselösung mit weiteren Komponenten bzw. Konzentraten oder auch in ein Bilanzkammersystem der Dialysemaschine gefördert werden.

Weitere aus dem Stand der Technik bekannte Dialysemaschinen finden sich in der US 2013/025692 A1, der US 2014/174542 A1, der US 2011/ 120302 A1 und der US 2013/028788 A1.

Darüber hinaus weisen bekannte Dialysemaschinen ein Pneumatiksystem auf, in dem zumindest zeitweise Vakuum herrscht. Das Vakuum wird von einem Kompressor des Pneumatiksystems erzeugt. Mit Druckluft und Vakuum werden z.B. Aktoren, wie beispielsweise Kolben und Zylinder betrieben. Auch ist es möglich, das erzeugte Vakuum z.B. zur Unterstützung einer Druckmessung zu nutzen.

Im Unterdruck sind die Dichtigkeitsanforderungen häufig höher und gleichzeitig aufwendiger umzusetzen, als in Überdrucksystemen. Somit muss das genannte Pneumatiksystem entsprechend aufwendig hergestellt werden bzw. mit aufwendigen Komponenten hergestellt werden, um eine hinreichende Dichtigkeit und somit ein zuverlässiges Halten des Unterdrucks realisieren zu können.

In Dialysemaschinen mit einem derartigen Pneumatiksystem spielt das durch den laufenden bzw. anlaufenden Kompressor des Pneumatiksystems erzeugte Geräusch insofern eine Rolle, als dass die Patienten und das klinische Personal diesen Geräuschen meist von mehreren Geräten gleichzeitig ausgesetzt sind.

Vor diesem Hintergrund wird angestrebt, die Laufzeiten sowie die Anzahl der Anlaufzyklen des Kompressors so gering wie möglich zu halten, was allerdings erfordert, dass die Dichtigkeitsanforderung für das gesamte Unterdrucksystem des Pneumatiksystems entsprechend weiter erhöht sind, was kostenintensiv ist.

Die Laufzeiten sowie die Anzahl der Anlaufzyklen des Kompressors spielen auch bezüglich der Lebensdauer des Kompressors und somit auch bezüglich der Kosten bei der auszuwählenden Kompressorkomponente eine wesentliche Rolle.

Somit ist es vorteilhaft, die Laufzeiten und Anlaufzyklen des Kompressors aus Kosten- und Geräuschaspekten so niedrig wie möglich zu halten. Eine weitere Anforderung besteht darin, dass die Dichtigkeit des Pneumatiksystems an allen Dichtigkeitsstellen nicht über das ohne besondere Maßnahmen, wie z.B. die Auswahl von Pneumatikbauteilen, erreichbare Maß erhöht wird. Dies hat unmittelbaren Einfluss auf die Komponentenkosten und über die Lebensdauer auch über die Verfügbarkeit sowie auf die Reparaturkosten.

Der vorliegenden Erfindung liegt somit die Aufgabe zugrunde, eine Dialysemaschine der eingangs genannten Art dahingehend weiterzubilden, dass eine Vakuumerzeugung im Pneumatiksystem mit geringem Kosten- und Geräuschaufwand möglich ist.

Diese Aufgabe wird durch eine Dialysemaschine mit den Merkmalen des Anspruchs 1 gelöst. Danach ist vorgesehen, dass zwischen der Saugseite der Entgasungspumpe und dem Pneumatiksystem zur Unterdruckerzeugung im Pneumatiksystem zumindest eine Verbindungsleitung existiert.

Durch diese Verbindungsleitung, die mit der Saugseite der Entgasungspumpe des Hydrauliksystems in Verbindung steht ist es möglich, eine Unterdruckerzeugung im Pneumatiksystem zu erreichen, ohne dass dazu ein separater Kompressor im Pneumatiksystem erforderlich ist.

Der Begriff "Saugseite der Entgasungspumpe" ist weit zu fassen und umfasst nicht nur beispielsweise die zur Pumpe führende Leitung, sondern auch andere Komponenten, die mit der Saugseite der Entgasungspumpe in Verbindung stehen, wie beispielsweise den Entgasungsbehälter etc.

Die Vakuumerzeugung, die über die gesamte Behandlungszeit, jedenfalls während der Betriebszeit der Entgasungspumpe des Hydrauliksystems verfügbar ist, kann somit mittels einer ohnehin laufenden bzw. ohnehin vorhandenen Pumpe (Entgasungspumpe) zur Verfügung gestellt werden. Zusätzliche Geräusche entstehen dadurch nicht und das Vakuum steht auch bei erhöhter Leckage in ausreichendem Maße zur Verfügung.

Somit erhöht sich insgesamt der Komfort einer solchen Dialysemaschine, da das Kompressorgeräusch entfallen kann. Die Kosten der Dialysemaschine werden durch die Verwendung von Standardkomponenten bei gleichzeitiger Erhöhung der Verfügbarkeit gesenkt.

Vorzugsweise ist vorgesehen, dass die Verbindungsleitung ansteuerbar ist, worunter zu verstehen ist, dass sich in der Verbindungsleitung zumindest ein Ventil oder ein sonstiges Absperrelement befindet, mittels dessen die Verbindungsleitung geöffnet und geschlossen sowie ggf. auch gedrosselt werden kann.

Besteht kein Bedarf einer weiteren Vakuumerzeugung im Pneumatiksystem, kann das Ventil geschlossen werden oder bleiben, soll jedoch eine Vakuumerzeugung stattfinden, wird das Ventil vorzugsweise bei laufender Entgasungspumpe geöffnet, sodass mittels der Entgasungspumpe eine Unterdruckerzeugung im Pneumatiksystem erfolgt.

Denkbar ist es, dass sich im Pneumatiksystem ein Drucksensor befindet und dass die Dialysemaschine eine Steuer- oder Regelungseinheit aufweist, die so ausgebildet ist, dass diese die Entgasungspumpe und/oder das in der Verbindungsleitung befindliche Ventil in Abhängigkeit des gemessenen Druckwertes betätigt. So ist es möglich, den Druck im Pneumatiksystem auf einem Sollwert zu erhalten oder zumindest innerhalb eines gewünschten Bereiches.

Vorzugsweise ist vorgesehen, dass das Pneumatiksystem abweichend von bekannten Dialysemaschinen keinen Kompressor zur Erzeugung eines Unterdrucks im Pneumatiksystem aufweist. Dies bringt Vorteile hinsichtlich der Kosten, der Wartungsintensität sowie auch der Geräuschkulisse der Dialysemaschine mit sich.

In diesem Fall wird der Unterdruck im Pneumatiksystem ausschließlich durch die Entgasungspumpe des Hydrauliksystems erzeugt. Grundsätzlich sind von der Erfindung jedoch auch Ausführungsformen umfasst, bei denen das Pneumatiksystem einen derartigen Kompressor zur Unterstützung der Vakuumerzeugung aufweist.

Bei der durch den Unterdruck zu betätigenden Komponente kann es sich beispielsweise um einen beliebigen Aktor, wie beispielsweise um einen Kolben oder Zylinder handeln. Auch ist es denkbar, dass das Vakuum in dem Pneumatiksystem zur Unterstützung einer Druckmessung genutzt wird. Der genannte Kolben oder Zylinder kann dazu betätigt werden, Flüsse innerhalb einer Kassette für die Dialyselösung oder auch Flüsse durch ein Leitungssystem z.B. für die Dialyselösung oder zulaufendes Wasser etc. zu steuern.

Denkbar ist es, dass das Hydrauliksystem wenigstens eine Entgasungskammer aufweist und dass ein Druckmesser zur Messung des Drucks in der Entgasungskammer vorgesehen ist. Die Dialysemaschine kann eine Steuer- oder Regelungseinheit aufweisen, die so ausgebildet ist, dass diese das Ventil der Abhängigkeit des gemessenen Druckes ansteuert.

Wie eingangs ausgeführt ist vorzugsweise vorgesehen, dass frisches Wasser bzw. RO-Wasser, das zur Herstellung der Dialyselösung benötigt wird in eine Entgasungskammer geleitet wird. In der Entgasungskammer wird durch die Entgasungspumpe ein Unterdruck erzeugt, wodurch eine Entgasung des Wassers erfolgt. Der gasförmige Anteil wird abgezogen und der flüssige Anteil wird mittels der Entgasungspumpe z.B. in eine Mischkammer befördert oder auch in ein Bilanzkammersystem der Dialysemaschine. Die Mischkammer kann so ausgebildet sein, dass das entgaste Wasser z.B. mit einem Konzentrat zur Herstellung der fertigen Dialyselösung gemischt wird.

Wird das Ventil in Abhängigkeit des gemessenen Druckes in der Entgasungskammer angesteuert bzw. geregelt, kann dadurch erreicht werden, dass der Druck in der Entgasungskammer nicht zu stark beeinflusst wird bzw. nicht unter einen Grenzwert fällt. Somit kann verhindert werden, dass der Druck in der Entgasungskammer einen unteren Grenzwert unterschreitet und es kann sichergestellt werden, dass der Druck in der Entgasungskammer innerhalb eines bestimmten Sollwertbereiches verbleibt, um die Entgasung des Wassers zuverlässig sicherzustellen.

Denkbar ist es somit, dass die Öffnungszeit des Ventils begrenzt wird, sodass der Druck in der Entgasungskammer nicht zu stark beeinflusst bzw. gesenkt wird.

In einer Ausgestaltung der Erfindung ist vorgesehen, dass die Dialysemaschine ein Bilanzkammersystem zur Bilanzierung des einem Dialysator der Dialysemaschine zugeführten und von diesem abgeführten Flüssigkeitsvolumens aufweist. Dieses Bilanzkammersystem dient beispielsweise zur Steuerung bzw. Einstellung des Ultrafiltrationsvolumens bzw. der Ultrafiltrationsrate. Des Weiteren kann die Dialysemaschine eine Steuer- oder Regelungseinheit aufweisen, die so ausgebildet ist, dass diese das Ventil in der wenigstens einen Verbindungsleitung in Abhängigkeit des Füllungsgrades des Bilanzkammersystems ansteuert.

So ist es denkbar, dass nur dann eine Vakuumerzeugung im Pneumatiksystem durchgeführt wird, wenn die Bilanzkammer vollständig gefüllt ist. Dazu wird der Zustand der Bilanzkammer ausgewertet und während der Füllphase das Ventil geschlossen gehalten. Ist die Bilanzkammer gefüllt, kann das Ventil bei Bedarf von Unterdruck im Pneumatiksystem geöffnet werden.

Als weitere Ausgestaltung der Erfindung ist vorgesehen, dass die Förderleistung, beispielsweise die Drehzahl der Entgasungspumpe veränderlich ist.

So ist es möglich, dass die Dialysemaschine eine Steuer- oder Regelungseinheit aufweist, die derart ausgebildet ist, dass diese die Förderleistung der Entgasungspumpe in Abhängigkeit der Ventilstellung steuert oder regelt.

So ist es denkbar, dass die Drehzahl der Entgasungspumpe bzw. deren Förderleistung während der Phasen der Vakuumerzeugung im Pneumatiksystem erhöht wird, um die Vakuumerzeugung im Pneumatiksystem einerseits zu beschleunigen und andererseits den Druck in der Entgasungskammer nicht zu stark zu beeinflussen bzw. abzusenken.

Wird somit das Ventil geöffnet, um Vakuum in dem Pneumatiksystem zu erzeugen, kann vorgesehen sein, dass für diese Zeitspanne, für das Ventil offen steht, die Förderrate der Entgasungspumpe erhöht wird. Als weitere Option kann das Ventil kurzzeitig geöffnet werden. Es kann intervallweise geöffnet und geschlossen werden, um in dem Pneumatiksystem einen Unterdruck zu erzeugen bzw. einen bestimmten Unterdruckwert oder Bereich zu erhalten.

Die vorliegende Erfindung betrifft des Weiteren ein Verfahren zum Betreiben eines Pneumatiksystems einer erfindungsgemäßen Dialysemaschine, wobei das Verfahren den Schritt umfasst, dass der Unterdruck in dem Pneumatiksystem ausschließlich oder auch durch die Entgasungspumpe des Hydrauliksystems der Dialysemaschine erzeugt wird.

Vorzugsweise ist vorgesehen, dass der Unterdruck ausschließlich durch die Entgasungspumpe des Hydrauliksystems erzeugt wird, sodass auf ein Unterdruckerzeugungsmittel, insbesondere auf einen Kompressor für das Pneumatiksystem verzichtet werden kann.

Wie oben ausgeführt, kann die Verbindungsleitung zwischen der Saugseite der Entgasungspumpe und dem Pneumatiksystem je nach Bedarf durch ein Ventil geschlossen oder geöffnet werden.

Denkbar ist es, dass die Dialysemaschine ein Bilanzkammersystem aufweist und dass das Ventil während der Füllphase des Bilanzkammersystems geschlossen bleibt.

Alternativ oder zusätzlich dazu kann vorgesehen sein, dass die Dialysemaschine eine Entgasungskammer aufweist, die auf der Saugseite der Entgasungspumpe angeordnet ist und dass das Ventil in Abhängigkeit des in der der Entgasungskammer herrschenden Druckes geöffnet oder geschlossen wird. Auf diese Weise ist es möglich, in der Entgasungskammer einen bestimmten Druckwert bzw. einen bestimmten Druckbereich zu halten bzw. zu verhindern, dass der Druck in der Entgasungskammer einen unteren Grenzwert unterschreitet.

Die Förderleistung der Entgasungspumpe kann während der Phasen der Unterdruckerzeugung im Pneumatiksystem erhöht werden. Dadurch ist es möglich, zu verhindern, dass der Druck in der Entgasungskammer zu stark abfällt und gleichzeitig wird dadurch eine rasche Unterdruckerzeugung im Pneumatiksystem erreicht.

In einer Option kann das Ventil kurzzeitig geöffnet werden. Denkbar ist ein intervallweise schließendes und öffnendes Ventil, um die Unterdruckerzeugung im Pneumatiksystem zu erreichen.

Aufgrund der Tatsache, dass die Vakuumerzeugung aufgrund der ohnehin vorhandenen Entgasungspumpe bereits vorhanden ist, spielen Häufigkeit und Dauer der Druckaufbauphasen eine untergeordnete Rolle und die Anforderungen an die innere Dichtigkeit der Pneumatikventile bzw. sonstigen Bestandteile des Pneumatiksystems können reduziert werden. Auch kann das Vakuumreservoir verkleinert werden, was weitere Einsparungen ermöglicht.

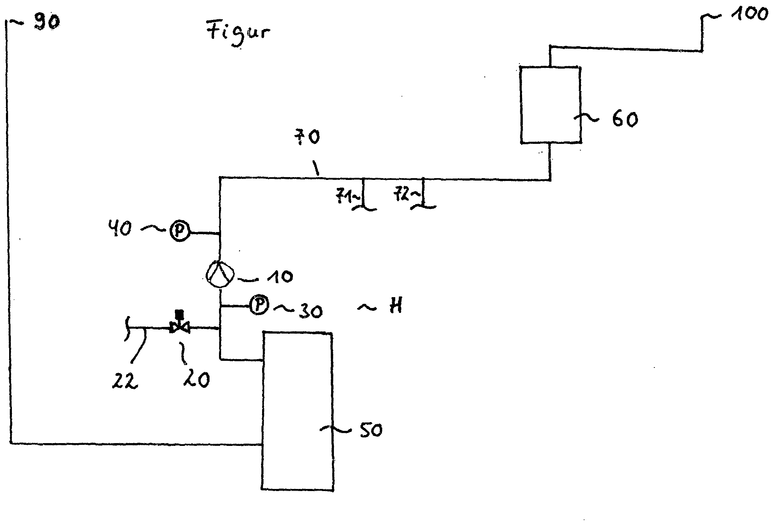

Weitere Einzelheiten und Vorteile der Erfindung werden anhand eines in der Zeichnung dargestellten Ausführungsbeispiels näher erläutert.

Die einzige Figur zeigt in einer schematischen Darstellung einen Teil des Hydrauliksystems einer erfindungsgemäßen Dialysemaschine.

Das Bezugszeichen 90 kennzeichnet einen Wasserzulauf des mit dem Bezugszeichen H gekennzeichneten Hydrauliksystems einer Dialysemaschine.

Das Wasser wird benötigt, um aus diesem eine Dialyselösung herzustellen, die dem Bilanzkammersystem 60 zugeführt wird. Das Bezugszeichen 100 kennzeichnet den Anschluss, über den die fertige Dialyselösung einem nicht dargestellten Dialysator der Dialysemaschine zugeführt wird.

Der Dialysator weist zwei Kammern auf, die durch eine oder mehrere semipermeable Membranen, vorzugsweise in Form einer Vielzahl von Hohlfasern, die semipermeable Wandungen aufweisen, voneinander getrennt sind. Die Dialysatkammer wird von der hergestellten Dialyselösung durchströmt und die Blutkammer von dem Blut des Patienten, das einen extrakorporalen Kreislauf durchströmt.

Das zulaufende Wasser 90 gelangt in den Entgasungsbehälter 50, in dem mittels der Entgasungspumpe 10 ein Unterdruck erzeugt wird.

Die Entgasungspumpe 10 fördert das entgaste Wasser in die Leitung 70. Dort werden über Zulaufleitungen 71, 72 Konzentrate zugegeben, die mit dem Wasser gemischt werden, um auf diese Weise die fertige Dialyselösung herzustellen. Diese wird dann dem Bilanzkammersystem 60 und von diesem der Dialysatseite des Dialysators zugeführt.

Wie dies aus der Figur weiter hervorgeht, erstreckt sich von der Saugseite bzw. von dem saugseitigen Zulauf der Entgasungspumpe 10 die Verbindungsleitung 22, in der sich ein Absperrventil 20 befindet. Das Absperrventil weist eine Antriebseinheit auf, mittels derer das Ventil geöffnet und geschlossen werden kann.

Die Leitung 22 verläuft zu einem in der Figur nicht dargestellten Pneumatiksystem der Dialysemaschine, in dem ein Unterdruck herrscht. Die Bezugszeichen 30 und 40 kennzeichnen Sensoren zur Messung des Druckes vor und nach der Entgasungspumpe 10. Diese Sensoren 30, 40 sind nicht zwingenderweise Bestandteil der Dialysemaschine, sondern dienen dem hier dargestellten Ausführungsbeispiel zur Überprüfung der Druckverhältnisse bei einem Druckhaltetest oder bei der Vorbereitung der Dialyselösung.

Wird in dem Pneumatiksystem Unterdruck benötigt, wird vorzugsweise bei laufender Entgasungspumpe 10 das Ventil 20 geöffnet und somit durch die Entgasungspumpe 10 das Vakuum auch im Pneumatiksystem erzeugt.

Ein zusätzlicher und eigens für die Vakuumerzeugung vorgesehener Kompressor ist im Pneumatiksystem nicht vorgesehen. Dadurch ergeben sich Geräusch-, Kosten- und Wartungsvorteile.

Versuche haben gezeigt, dass die Vakuumerzeugungsleistung durch die Entgasungspumpe 10 ausreichend ist, um während der Vorbereitung der Dialysebehandlung einen hinreichenden Unterdruck im Pneumatiksystem aufrecht zu erhalten.

Der Unterdruckzweig des Pneumatiksystems wurde mit dem Hydrauliksystem H der Dialysemaschine über die Leitung 22 bei offenem Ventil 20 verbunden. Während der Vorbereitungsphase der Dialysebehandlung stieg der erzeugte Unterdruck nie über einen bestimmten Grenzwert, d.h. es konnte während der Vorbereitung der Behandlung stets ein gewünschtes Unterdruckniveau gehalten werden.

Bei einem Druckhaltetest konnte gezeigt werden, dass durch das Hydrauliksystem bzw. durch die Entgasungspumpe 10 im Pneumatiksystem ein ausreichender Unterdruck erzeugt werden kann. So konnte bei konstanter Drehzahl der Entgasungspumpe in relativ kurzer Zeit ein ausreichendes Unterdruckniveau im Pneumatiksystem erzielt werden, das es erlaubt, beispielsweise Aktoren etc. zu betätigen.

Durch eine Steigerung der Vakuumerzeugungsleistung durch die Steigung der Drehzahl der Entgasungspumpe 10 konnte gezeigt werden, dass binnen noch kürzerer Zeit ein ausreichend hohes Vakuum im Pneumatiksystem erzielt werden konnte.

Wie bereits oben ausgeführt, ist es nicht zwingend erforderlich, dass die Verbindungsleitung 22 mit der saugseitigen Leitung der Pumpe 10 verbunden ist. Auch ist es denkbar, dass die Verbindungsleitung beispielsweise in den Entgasungsbehälter 50 mündet, in dem Vakuum herrscht.

Die Verbindungsleitung kann unmittelbar oder auch mittelbar mit der Saugseite der Entgasungspumpe verbunden sein.

Durch die vorliegende Erfindung ist es möglich; eine Dialysemaschine bereitzustellen, die es erlaubt, mit vergleichsweise geringem Kostenaufwand, Wartungsaufwand und geringerer Geräuschkulisse im Pneumatiksystem ein hinreichendes Vakuum während der Vorbereitung und Durchführung einer Dialysebehandlung bereitzustellen.

## Patentansprüche

1. Dialysemaschine mit einem Hydrauliksystem (H), das zur Bereitstellung der Dialyselösung oder eines Bestandteils der Dialyselösung bestimmt ist, wobei das Hydrauliksystem (H) eine Entgasungspumpe (10) zur Entgasung der Dialyselösung oder eines Bestandteils der Dialyselösung aufweist, sowie mit einem Pneumatiksystem, in dem zum Betrieb einer Komponente des Dialysegerätes zumindest zeitweise Unterdruck herrscht,
**dadurch gekennzeichnet,**
**dass** zwischen der Saugseite der Entgasungspumpe (10) und dem Pneumatiksystem zur Unterdruckerzeugung im Pneumatiksystem eine Verbindungsleitung (22) existiert, so dass mittels der Entgasungspumpe (10) eine Unterdruckerzeugung im Pneumatiksystem erfolgt.

2. Dialysemaschine nach Anspruch 1, **dadurch gekennzeichnet, dass** sich in der Verbindungsleitung ein Ventil befindet, durch das die Verbindungsleitung geöffnet und geschlossen werden kann.

3. Dialysemaschine nach Anspruch 1 oder 2, **dadurch gekennzeichnet, dass** sich in dem Pneumatiksystem ein Drucksensor befindet und dass die Dialysemaschine eine Steuer- oder Regelungseinheit aufweist, die derart ausgebildet ist, dass diese die Entgasungspumpe und/oder das in der Verbindungsleitung befindliche Ventil in Abhängigkeit des durch den Drucksensor gemessenen Wertes betätigt.

4. Dialysemaschine nach einem der vorhergehenden Ansprüche, **dadurch gekennzeichnet, dass** das Pneumatiksystem keinen Kompressor zur Erzeugung eines Unterdrucks im Pneumatiksystem aufweist.

5. Dialysemaschine nach einem der vorhergehenden Ansprüche, **dadurch gekennzeichnet, dass** es sich bei der durch den Unterdruck zu betätigenden Komponente um einen Aktor, insbesondere um einen Kolben oder Zylinder handelt.

6. Dialysemaschine nach einem der Ansprüche 2 bis 5, **dadurch gekennzeichnet, dass** das Hydrauliksystem eine Entgasungskammer aufweist und dass ein Druckmesser zur Messung des Druckes in der Entgasungskammer vorgesehen ist, und dass die Dialysemaschine eine Steuer- oder Regelungseinheit aufweist, die derart ausgebildet ist, dass diese das Ventil in Abhängigkeit des gemessenen Druckes ansteuert.

7. Dialysemaschine nach einem der Ansprüche 2 bis 6, **dadurch gekennzeichnet, dass** die Dialysemaschine ein Bilanzkammersystem zur Bilanzierung des einem Dialysator zugeführten und von diesem abgeführten Flüssigkeitsvolumens aufweist und dass die Dialysemaschine eine Steuer- oder Regelungseinheit aufweist, die derart ausgebildet ist, dass diese das Ventil in Abhängigkeit des Füllungsgrades des Bilanzkammersystems ansteuert.

8. Dialysemaschine nach einem der Ansprüche 2 bis 7, **dadurch gekennzeichnet, dass** die Förderleistung der Entgasungspumpe veränderlich ist.

9. Dialysemaschine nach Anspruch 8, **dadurch gekennzeichnet, dass** die Dialysemaschine eine Steuer- oder Regelungseinheit aufweist, die derart ausgebildet ist, dass diese die Förderleistung der Entgasungspumpe in Abhängigkeit der Ventilstellung steuert oder regelt.

10. Dialysemaschine nach einem der Ansprüche 2 bis 9, **dadurch gekennzeichnet, dass** die Dialysemaschine eine Steuer- oder Regelungseinheit aufweist, die derart ausgebildet ist, dass diese das Ventil zum Aufbau des Unterdrucks in dem Pneumatiksystem intervallweise öffnet und schließt.

11. Verfahren zum Betreiben eines Pneumatiksystems einer Dialysemaschine nach einem der Ansprüche 1 bis 10, wobei der Unterdruck in dem Pneumatiksystem ausschließlich oder auch durch die Entgasungspumpe des Hydrauliksystems der Dialysemaschine erzeugt wird.

12. Verfahren nach Anspruch 11, **dadurch gekennzeichnet, dass** die Verbindungsleitung zwischen der Saugseite der Entgasungspumpe und dem Pneumatiksystem je nach Bedarf durch ein Ventil geschlossen oder geöffnet wird.

13. Verfahren nach Anspruch 12, **dadurch gekennzeichnet, dass** die Dialysemaschine ein Bilanzkammersystem aufweist und dass das Ventil während der Füllphase des Bilanzkammersystems geschlossen bleibt und/oder dass die Dialysemaschine eine Entgasungskammer aufweist und dass das Ventil in Abhängigkeit des in der Entgasungskammer herrschenden Druckes geöffnet oder geschlossen wird.

14. Verfahren nach einem der Ansprüche 11 bis 13, **dadurch gekennzeichnet, dass** die die Förderleistung der Entgasungspumpe während der Phasen der Unterdruckerzeugung im Pneumatiksystem erhöht wird.

15. Verfahren nach einem der Ansprüche 12 bis 14, **dadurch gekennzeichnet, dass** das Ventil in der Entgasungsleitung intervallweise geschlossen und geöffnet wird.

## Claims

1. A dialysis machine having a hydraulic system (H) that is intended for providing a dialysis solution or an element of the dialysis solution, wherein the hydraulic system (H) has a degassing pump (10) for degassing the dialysis solution or an element of the dialysis solution, and having a pneumatic system in which a vacuum is present at least at times for operating a component of the dialysis machine,
**characterized in that**
there is a connection line (22) between the suction side of the degassing pump (10) and the pneumatic system for vacuum generation in the pneumatic system so that a vacuum generation takes place in the pneumatic system by means of the degassing pump (10).

2. A dialysis machine in accordance with claim 1, **characterized in that** a valve by which the connection line can be opened and closed is present in the connection line.

3. A dialysis machine in accordance with claim 1 or claim 2, **characterized in that** there is a pressure sensor in the pneumatic system; and **in that** the dialysis machine has a control or regulation unit that is configured such that it actuates the degassing pump and/or the valve located in the connection line in dependence on the value measured by the pressure sensor.

4. A dialysis machine in accordance with one of the preceding claims, **characterized in that** the pneumatic system does not have a compressor for generating a vacuum in the pneumatic system.

5. A dialysis machine in accordance with one of the preceding claims, **characterized in that** the component to be actuated by the vacuum is an actuator, in particular a piston or a cylinder.

6. A dialysis machine in accordance with one of the claims 2 to 5, **characterized in that** the hydraulic system has a degassing chamber; and **in that** a pressure gauge for measuring the pressure in the degassing chamber is provided; and **in that** the dialysis machine has a control or regulation unit that is configured such that it controls the valve in dependence on the measured pressure.

7. A dialysis machine in accordance with one of the claims 2 to 6, **characterized in that** the dialysis machine has a balancing chamber for balancing the fluid volume supplied to a dialyzer and led off therefrom; and **in that** the dialysis machine has a control or regulation unit that is configured such that it controls the valve in dependence on the degree of filling of the balancing chamber system.

8. A dialysis machine in accordance with one of the claims 2 to 7, **characterized in that** the pump capacity of the degassing pump is variable.

9. A dialysis machine in accordance with claim 8, **characterized in that** the dialysis machine has a control or regulation unit that is configured such that it controls or regulates the pump capacity of the degassing pump in dependence on the valve position.

10. A dialysis machine in accordance with one of the claims 2 to 9, **characterized in that** the dialysis machine has a control or regulation unit that is configured such that it opens and closes the valve for building up the vacuum in the pneumatic system at intervals.

11. A method of operating a pneumatic system of a dialysis machine in accordance with one of the claims 1 to 10, wherein the vacuum in the pneumatic system is generated only or also by the degassing pump of the hydraulic system of the dialysis machine.

12. A method in accordance with claim 11, **characterized in that** the connection line between the suction side of the degassing pump and the pneumatic system is closed or opened by a valve as required.

13. A method in accordance with claim 12, **characterized in that** the dialysis machine has a balancing chamber system; and **in that** the valve remains closed during the filling phase of the balancing chamber system; and/or **in that** the dialysis machine has a degassing chamber; and **in that** the valve is opened or closed in dependence on the pressure present in the degassing chamber.

14. A method in accordance with one of the claims 11 to 13, **characterized in that** the pump capacity of the degassing pump is increased during the phases of vacuum generation in the pneumatic system.

15. A method in accordance with one of the claims 12 to 14, **characterized in that** the valve is closed and opened at intervals in the degassing line.

## Revendications

1. Machine de dialyse dotée d'un système hydraulique (H), qui est destiné à mettre à disposition la solution de dialyse ou un constituant de la solution de dialyse, le système hydraulique (H) comportant une pompe de dégazage (10) pour le dégazage de la solution de dialyse ou d'un constituant de la solution de dialyse, ainsi que d'un système pneumatique, dans lequel règne au moins temporairement une dépression pour faire fonctionner un composant de l'appareil de dialyse,
**caractérisée en ce que**,
entre le côté aspiration de la pompe de dégazage (10) et le système pneumatique, se trouve une conduite de liaison (22) pour générer la dépression dans le système pneumatique, de telle sorte qu'une dépression est générée dans le système pneumatique au moyen de la pompe de dégazage (10).

2. Machine de dialyse selon la revendication 1, **caractérisée en ce qu'**une vanne, par laquelle la conduite de liaison peut être ouverte et fermée, se trouve dans la conduite de liaison.

3. Machine de dialyse selon la revendication 1 ou 2, **caractérisée en ce qu'**un capteur de pression se trouve dans le système pneumatique et **en ce que** la machine de dialyse comporte une unité de commande ou de régulation, qui est conçue de manière à actionner la pompe de dégazage et/ou la vanne se trouvant dans la conduite de liaison en fonction de la valeur mesurée par le capteur de pression.

4. Machine de dialyse selon l'une des revendications précédentes, **caractérisée en ce que** le système pneumatique ne comporte pas de compresseur pour générer une dépression dans le système pneumatique.

5. Machine de dialyse selon l'une des revendications précédentes, **caractérisée en ce que** le composant à actionner par la dépression est un actionneur, en particulier un piston ou un cylindre.

6. Machine de dialyse selon l'une des revendications 2 à 5, **caractérisée en ce que** le système hydraulique comporte une chambre de dégazage et **en ce qu'**un manomètre est prévu pour mesurer la pression dans la chambre de dégazage, et **en ce que** la machine de dialyse comporte une unité de commande ou de régulation, qui est conçue de manière à commander la vanne en fonction de la pression mesurée.

7. Machine de dialyse selon l'une des revendications 2 à 6, **caractérisée en ce que** la machine de dialyse comporte un système de chambre d'équilibrage pour la mise en équilibre du volume de liquide alimenté dans un dialyseur et évacué de celui-ci et **en ce que** la machine de dialyse comporte une unité de commande ou de régulation, qui est conçue de manière à commander la vanne en fonction du degré de remplissage du système de chambre d'équilibrage.

8. Machine de dialyse selon l'une des revendications 2 à 7, **caractérisée en ce que** la capacité de pompage de la pompe de dégazage est variable.

9. Machine de dialyse selon la revendication 8, **caractérisée en ce que** la machine de dialyse comporte une unité de commande ou de régulation, qui est conçue de manière à commander ou réguler la capacité de pompage de la pompe de dégazage en fonction de la position de la vanne.

10. Machine de dialyse selon l'une des revendications 2 à 9, **caractérisée en ce que** la machine de dialyse comporte une unité de commande ou de régulation, qui est conçue de manière à ouvrir et fermer par intermittence la vanne pour établir la dépression dans le système pneumatique.

11. Procédé pour faire fonctionner un système pneumatique d'une machine de dialyse selon l'une des revendications 1 à 10, dans lequel la dépression dans le système pneumatique est générée exclusivement ou également par la pompe de dégazage du système hydraulique de la machine de dialyse.

12. Procédé selon la revendication 11, **caractérisé en ce que** la conduite de liaison entre le côté aspiration de la pompe de dégazage et le système pneumatique est fermée ou ouverte par une vanne selon les besoins.

13. Procédé selon la revendication 12, **caractérisé en ce que** la machine de dialyse comporte un système de chambre d'équilibrage et **en ce que** la vanne reste fermée pendant la phase de remplissage du système de chambre d'équilibrage et/ou **en ce que** la machine de dialyse comporte une chambre de dégazage et **en ce que** la vanne est ouverte ou fermée en fonction de la pression régnant dans la chambre de dégazage.

14. Procédé selon l'une des revendications 11 à 13, **caractérisé en ce que** la capacité de pompage de la pompe de dégazage est augmentée pendant les phases de génération de la dépression dans le système pneumatique.

15. Procédé selon l'une des revendications 12 à 14, **caractérisé en ce que** la vanne dans la conduite de dégazage est fermée et ouverte par intermittence.
